# EUROPEAN PATENT SPECIFICATION

(11) **EP 1 242 086 B1**
(45) Date of publication and mention of the grant of the patent: **05.10.2005**
(21) Application number: 00985615.4
(22) Date of filing: 18.12.2000
(51) Int. Cl.: A61K 31/473, A61K 31/381, A61K 31/343, A61P 37/00, A61P 37/06

(54) **CD45 INHIBITORS**
CD45 HEMMER
INHIBITEURS DE CD45

(30) Priority: 21.12.1999 US 172785 P
(43) Date of publication of application: 25.09.2002
(73) Proprietor: AstraZeneca AB, 151 85 Södertälje (SE)
(72) Inventor: CHAPDELAINE, Marc, Jerome, Wilmington, DE 19850-5437 (US); KNAPPENBERGER, Katherine, Wilmington, DE 19850-5437 (US); STEELMAN, Gary, Wilmington, DE 19850-5437 (US); SUCHARD, Suzanne, Wilmington, DE 19850-5437 (US); SYGOWSKI, Linda, Wilmington, DE 19850-5437 (US); URBANEK, Rebecca, Wilmington, DE 19850-5437 (US); VEALE, Chris, Allan, Wilmington, DE 19850-5437 (US)
(74) Representative: Bryant, Tracey
(86) International application number: PCT/GB2000/004867
(87) International publication number: WO 2001/045680

(56) References cited:
- URBANEK, REBECCA A. ET AL: "Potent Reversible Inhibitors of the Protein Tyrosine Phosphatase CD45" J. MED. CHEM. (2001), 44(11), 1777-1793 , XP001014517
- GOULART, MARFLIA O. F. ET AL: "Trypanocidal activity and redox potential of heterocyclic- and 2-hydroxy-naphthoquinones" BIOORG. MED. CHEM. LETT. (1997), 7(15), 2043-2048 , XP004136382
- RIBERIRO-RODRIGUES, R. ET AL: "Growth inhibitory effect of naphthofuran and naphthofuranquinone derivatives on Trypanosoma cruzi epimastigotes" BIOORG. MED. CHEM. LETT. (1995), 5(14), 1509-12 , XP004135435
- CLARK A.M. ET AL: "In vitro antimicrobial activity of benzoquinolinediones." PHARMACEUTICAL RESEARCH, (1984) NO. 6/- (269-271). CODEN: PHREEB, XP000908989
- J. BRAVEN ET AL.: "Synthetic Routes to Indenopyridine Analogues of Morphactins" JOURNAL OF HETEROCYCLIC CHEMISTRY, vol. 32, 1995, pages 1051-1055, XP001015632 cited in the application
- M.A.F. BRANDAO ET AL.: "Combined Directed Metalation-Cross Coupling Strategies" TETRAHEDRON LETTERS, vol. 34, no. 15, 1993, pages 2437-2440, XP001015633 cited in the application

## Description

### Background

### Field of the Invention

Compounds and methods for the treatment of autoimmune disorders and organ graft rejection.

### Related Art

Action of the immune system is known to be involved in immunologically-related diseases and disorders such as autoimmune disorders and in organ graft rejection ("OGR"). Hematopoietic, thymus-derived cells, (so-called "T cells") have an important and pervasive role as regulators and effectors of the functions of the immune system. Hematopoietic cells, and T cells in particular have on their surfaces a major transmembrane glycoprotein designated CD45, characterized by a cluster of antigenic determinants. CD45 is also known as leukocyte common antigen ("LCA"). The cytosolic portion of CD45 has protein tyrosine phosphatase ("PTP") activity and CD45 activity is known to be essential for TCR initiated T cell activation. Studies in CD45-deficient cell lines have shown that CD45 is a positive regulator of the T-Cell Receptor ("TCR") and that CD45 functions in TCR regulation by dephosphorylating the src kinases p56^{*lck*} and p59^{*fyn*}, which allows autophosphorylation of the positive regulatory site on these enzymes; these reactions lead to downstream events and ultimately to T cell activation.

Available treatments for autoimmune disorders and OGR have therapeutic disadvantages. For example, Cyclosporin A, the drug most commonly used to treat OGR, has renal and CNS toxicity.

### Summary of the Invention

Potent inhibitors of CD45 have been discovered. Such inhibitors are useful for the treatment of various autoimmune disorders as well as for treatment of OGR. Inhibition of the phosphatase activity of CD45 by compounds of the present invention has been shown by incubating the cytosolic portion of CD45 with the compounds and *p*-nitrophenyl phosphate (*p*NPP), a phosphatase substrate. Spectrophotometric monitoring has shown that the liberation of *p*-nitrophenol from the substrate by CD45 is inhibited in the presence of the compounds disclosed herein. Inhibition of the phosphatase activity of CD45 by compounds of the present invention has also been shown using a p56^{*lck*} carboxy-terminal phosphorylated peptide as a substrate. Compounds of the present invention have also been shown to inhibit proliferation ofT cells in a T-cell proliferation assay.

Compounds of the present invention are heterocyclic compounds selected from benzo[f]quinoline-5,6-dione; benzo[h]quinoline-5,6-dione; naphtho[1,2-b]furan-4,5-dione, and naphtho[1,2-b]thiophene-4,3-dione.

Compounds of the present invention are ligands of CD45 which, when bound, inhibit the activity of the protein tyrosine phosphatase (PTP) activity of the cytosolic portion of CD45. Binding of a compound of the present invention to CD45 inhibits the activity of CD45 essential for TCR initiated T cell activation. Thus, compounds of the invention inhibit the positive regulation of the TCR that leads to downstream events and T cell activation. Compounds of the present invention are useful to suppress the action of the immune system in disorders such as autoimmune disorders and organ graft rejection and to inhibit the action of T cells as functional regulators and effectors of the immune system.

The present invention also encompasses use of compounds described herein for the manufacture of a medicament for treating autoimmune disorders or organ graft rejection.

### Detailed Description of the Invention

### Examples

### Examples 1 to 4:

The compounds of examples 1, benzo[f]quinoline-5,6-dione, and 2, benzo[h]quinaline-5,6-dione, were made substantially as disclosed by Braven, J.; Hanson, R. W.; Smith, N. G. *J. Heterocyclic Chem*. **1995**, 32, 1051-1056. The compounds of examples 3, naphtho[1,2-b]furan-4,5-dione, and 4, naphtho[1,2-b]thiophene-4,5-dione, were made substantially as disclosed by Brandao, M. A. F.; deOliveira, A. B.; Snieckus, V. *Tetrahedron Lett*. **1993**, *34*, 2437-2440,.

### Assays for Biological Activity

### Method A:

### Phospharase assay using pNPP as substrate:

CD45 enzyme was obtained from BIOMOL (Plymouth Meeting, PA). Phosphatase activity was assayed in a buffer containing final concentrations of 25 mM imidazole at pH 7.0, 50 mM NaCl, 2.5 mM ethylenediaminetetraacetic acid ("EDTA"), 5 mM dithiothreitol ("DTT") and 10 µg/mL bovine serum albumin ("BSA") usingpNPP as a substrate. Compounds were tested in a range from 30 to 0.01 µM, with a final concentration of 1 or 5% dimethylsulfoxide ("DMSO"), depending on the compound solubility. Activity was measured by following the increase in absorbance at 405 nm using a SpectraMax Plus spectrophotometric plate reader (Molecular Devices, Sunnyvale, CA).

### Method B:

### Cytotoxicity Assay:

Calcein-AM (Molecular Probes, Eugene, OR) uptake, as a quantitative measure of cell viability, was used to evaluate the toxic effect of compounds on T cells. Briefly, PBMC were treated for 3-7 days with 3-10 µg/ml PHA, a potent T-cell mitogen, to preferentially expand the T-cell population. (Bradley, Linda M. *Cell Poliferation* in *Selected Methods in Cellular Immunology,* Eds. Mishell, B.B. and Shiigi, S.M., W.H. Freeman and Co., San Francisco, 1980.)

The T-cell lymphoblasts were purified by separation over Lymphoprep, plated at 2 x 10⁵/well in a round bottom 96-well plate containing RPMI with compound and incubated overnight at 37 °C in an incubator containing 5% CO₂. The dilution scheme and culture media were the same as those used in the T-cell proliferation assay. After the incubation period, cells were washed with Dulbecco's phosphate- buffered saline (D-PBS) and incubated with 1µM Calcein-AM for 30-45 min in D-PBS as described in the technical sheet provided with The LIVE/DEAD Viability/Cytotoxicity Kit from Molecular Probes. Percent viability was assessed on a fluorescent plate reader (excitation filter 485/20 nm; emission filter 530/25 nm) where the 100% control value is the fluorescence intensity observed in the absence of test compound.

### Method C:

### Phosphatase assay using lck 10-mer as substrate:

Phosphatase activity was assayed in 96 well plates in a buffer containing final concentrations of 25 mM HEPES at pH 7.2, 5 mM DTT and 10 µg/mL BSA, using the *lck* carboxy-terminal peptide TEGQpYQPQP as the substrate (Cho, H., Krishnaraj, R., Itoh, M., Kitas, E., Bannwarth, W., Saito, H., Walsh, C.T. 1993). Substrate specificities of catalytic fragments of protein tyrosine phosphatases (HPTPb, LAR, and CD45) toward the phosphotyrosylpeptide substrates and thiophosphotyrosylated peptides as inhibitors. (*Protein* *Science* 2:977-984). Compounds were tested in a range from 30 to 0.01 µM in a final concentration of 5% DMSO. Enzyme was incubated with substrate, with or without compound, at room temperature for 1.5 h. At the end of the incubation period, BIOMOL "Green Reagent" (BIOMOL, Plymouth Meeting, PA) was added to each well, the plates incubated at room temperature for 30 min and absorbance read at 620 nm.

### Method D:

### Cell isolation and T cell proliferation assay:

Whole blood was obtained from healthy human blood donors. Peripheral blood mononuclear cells ("PBMC") were isolated using Lymphoprep density-gradient centrifugation (Nycomed Amersham, Oslo, Norway), washed, counted and resuspended at 2 X 10⁶ cells/mL in RPMI 1640 medium containing glutamine, 0.1 mg/mL gentamycin and 10% heat inactivated human serum. PBMC were transferred to 96-well plates (2 X 10⁵ cells/well) containing compound or vehicle control, with the final concentration of DMSO not to exceed 0.3%, and incubated for 1 hour before addition of the activating anti-CD3 antibody, OKT3 (30 ng/mL). After 24 hours in culture, the cells were pulsed with [³H]thymidine (1 µCi/well) overnight and harvested the next day onto 96-well Packard GF/C filter plates using a Packard Cell Harvester (Packard Instruments, Meriden, CT). The filter plate was dried, the bottom of the plate sealed, 25 µL of Microscint 20 scintillation fluid added to each well, the top of the plate sealed with TopSeal-A, and the plate counted on a Packard TopCount. The data from the TopCount is transferred into Excel 5 (Microsoft, Redmond, WA) and formatted for EC₅₀ determination using Prism software (GraphPad Software, San Diego, CA).

Table 1 shows the inhibition of CD45 activity in the *p*NPP asssay and the *lck* assay certain compounds of the present invention. Inhibition in the T cell proliferation assay, as well as results from T cell cytotoxicity assay are shown.

**Table 1:**

| **Example. No.** | **pNPP IC**_{**50**} **(µM)** | **lck IC**_{**50**} **(µM)** | **T cell prolif. IC**_{**50**} **(µM)** | **CC**_{**50**} **(µM)** |
|---|---|---|---|---|
| **1** | 2.3 | >30 | 0.4 | 7 |
| **2** | 1.0 | 3.1 | 0.24 | 2.4 |
| **3** | 1.1 | 4.5 | 0.6 | 3.5 |
| **4** | 1.5 | >30 | 0.6 | 3.5 |

## Claims

1. Use of a compound selected from:
benzo[f]quinoline-5,6-dione;
benzo[h]quinoline-5,6-dione;
naphtho[1,2-b]furan-4,5-dione, and
naphtho[1,2-b]thiophene-4,5-dione,
or tautomers thereof or pharmaceutically-acceptable salts thereof, for the manufacture of a medicament for treating autoimmune disorders or organ graft rejection.

## Patentansprüche

1. Verwendung einer Verbindung, ausgewählt aus:
Benzo[f]chinolin-5,6-dion;
Benzo[h]chinolin-5,6-dion;
Naphtho[1,2-b]furan-4,5-dion und
Naphtho[1,2-b]thiophen-4,5-dion
oder Tautomeren davon oder pharmazeutisch annehmbaren Salzen davon, zur Herstellung eines Arzneimittels zur Behandlung von Autoimmunerkrankungen oder einer Organtransplantatabstoßungsreaktion.

## Revendications

1. Utilisation d'un composé choisi parmi :
la benzo[f]quinoléine-5,6-dione ;
la benzo[h]quinoléine-5,6-dione ;
la naphto[1,2-b]furane-4,5-dione, et
la naphto[1,2-b]thiophène-4,5-dione,
ou des tautomères de celles-ci ou des sels pharmaceutiquement acceptables de celles-ci, pour la fabrication d'un médicament destiné au traitement de troubles autoimmunes ou de rejet de greffes d'organes.
